# EUROPEAN PATENT SPECIFICATION

(11) **EP 1 158 296 B1**
(45) Date of publication and mention of the grant of the patent: **21.03.2007**
(21) Application number: 01500119.1
(22) Date of filing: 14.05.2001
(51) Int. Cl.: G01N 33/38, G01N 29/04, G01N 21/88, G01N 29/12, G01N 29/14, G06T 7/00

(54) **Machine for automated quality control of ceramic products by artificial vision and sound analysis**
Vorrichtung für die automatische Qualitätskontrolle keramischer Produkte durch künstliche Sicht- und Klanganalyse
Appareil pour la contrôle automatique de la qualité de produits céramiques par l'analyse optique artificielle et sonique

(30) Priority: 16.05.2000 ES 200001298
(43) Date of publication of application: 28.11.2001
(73) Proprietor: Ceramic Machine Vision, S.L., 08700 Igualada (Barcelona) (ES)
(72) Inventor: Segui Pascual, Vicente, 08700 Igualada (Barcelona) (ES)
(74) Representative: Carvajal y Urquijo, Isabel

(56) References cited:
- EP-A- 0 636 881
- DE-A- 10 100 467
- US-A- 5 285 687
- US-A- 5 699 153
- US-A- 5 894 345

## Description

### OBJECT OF THE INVENTION

The present invention relates to automated quality control of ceramic products, such as roof, wall and floor tiles, bricks and ceramic products in general.

Current quality control systems are manual and are performed by human operators, both for visual and sound control, which in certain operators causes eventual hearing problems after a number of years, excessive stress due to the large production of parts/hour and in addition a low reliability of the quality control.

### BACKGROUND OF THE INVENTION

Hitherto, control of defects in ceramic products is performed by visual inspection by operators, which entails a number of disadvantages and limitations.

Productions of manufactured parts / day in the ceramic industry are very high, and for roof and wall tiles may exceed 100,000 units/day. This requires the operators to control a large number of parts/hour, with the ensuing fatigue and decreased efficiency of the control after the first 2 hours of each shift.

In addition, in order to detect internal defects which are not visible, such as for ceramic roof tiles, the operator must strike each part with a hammer and by means of the sound produced, and after years of experience, the presence of any internal defects can be detected.

However, there are usually several inspection lines and several operators striking their part at the same time, which causes a sound interference of the sound made by one operator when striking the part with that of other operators, thereby hindering the correct appreciation of these internal defects. Over the years these operators also tend to suffer from various hearing problems.

Furthermore, as occurs with the visual inspection, their sense of hearing is dulled after a while, and so it is not possible to assure a given percentage of correct parts in those sent for packaging and sale, leading to occasional legal actions and claims between the manufacturer and the client.

For this reason, a system has been designed which is based on the principle of artificial or computerised vision and on sound analysis, which solves all of the above problems.

As prior art regarding the subject matter of the current invention, there is a document (US 5 285 687, Ringel Wemer *et al*) which describes a process as well as an device for the acoustic examination of monoliths for use in manufacturing of waste gas catalyst wherein a plurality of steps are comprised. One of this steps consists on performing synchronously at least two measurements of sound pressure.

On the other hand, the document US 5 699 153 discloses an optical inspecting method and apparatus therefor in order to detect an optical non uniformity in an object.

Advantageously, the object of the current invention achieves the integration of two systems for detecting external and internal defects of ceramic products by means of an only one machine.

### DESCRIPTION OF THE INVENTION

The system consists of a part transportation system comprising two belts which rest on two guide rails. The belts are driven through the corresponding pulleys by a motorised speed reducer with a frequency shifter so that the optimal speed can be achieved for each type of part.

Additionally, the distance between pulleys can be changed and is set according to the size of the controlled parts.

The first control area, the sound area, is meant to detect any possible internal defects of the part. It is provided with a hammer which strikes the part automatically when the system detects its presence. The sound wave created is sent to a computer where it is analysed, so that according to its acoustic spectrogram the system will classify it as either good or bad.

After this first control area the part enters the second control area, where it is inspected by the artificial vision system which sends the image to the computer, where it is processed for detection of visual defects and ensuring that its size is within the limits set for allowable tolerance.

The results of the artificial vision analysis allows detection of the following defects:
- Paint defects
- Incorrect colour hue
- Broken corners or wrong angles
- Incomplete part
- Size beyond tolerance limits
- Fissures

After the analyses of areas 1 and 2 are performed, the computer classifies the part as good or bad. If it is bad, it also displays the type of defect on the monitor.

If the part is bad the system automatically generates the electrical signal which causes the part to be expelled from the inspection line.

The monitor screen also has part counters which at all times provide the following information:
- Total number of parts controlled
- Total number of correct parts
- Total number of incorrect parts

In addition, there are other counters which classify incorrect parts according to the type of defect, such as:
- Total number of parts lacking material
- Total number of parts with sound defects
- Total number of parts with paint colour defects

The system is completed by a control cabin in which are housed the monitor, computer and printer, power sources and a programmable robot.

The system can also print out work shift, daily, weekly or monthly historical and statistical records.

The machine is also provided with a protection casing with sound insulation to avoid acoustic interferences from ambient noise. In addition the enclosure where the vision system is located is also insulated to eliminate influence of ambient light and dust.

The enclosure for the artificial vision system is pressurised to prevent entry of dust.

Lastly, the arm which connects control area number 2 to the cabin houses the control and command cables.

The system is provided with on or more black and white and/or colour cameras, depending on the type of control to be performed.

The cameras are positioned automatically by the computer, according to the type and size of the part inspected. This is done with a linear actuator driven by a servomotor and a programmable robot.

### BRIEF DESCRIPTION OF THE DRAWINGS

The characteristics of the invention will be made clearer in view of the accompanying drawings of a preferred embodiment of the machine for quality control of ceramic products, where for purposes of illustrations only the following is shown:

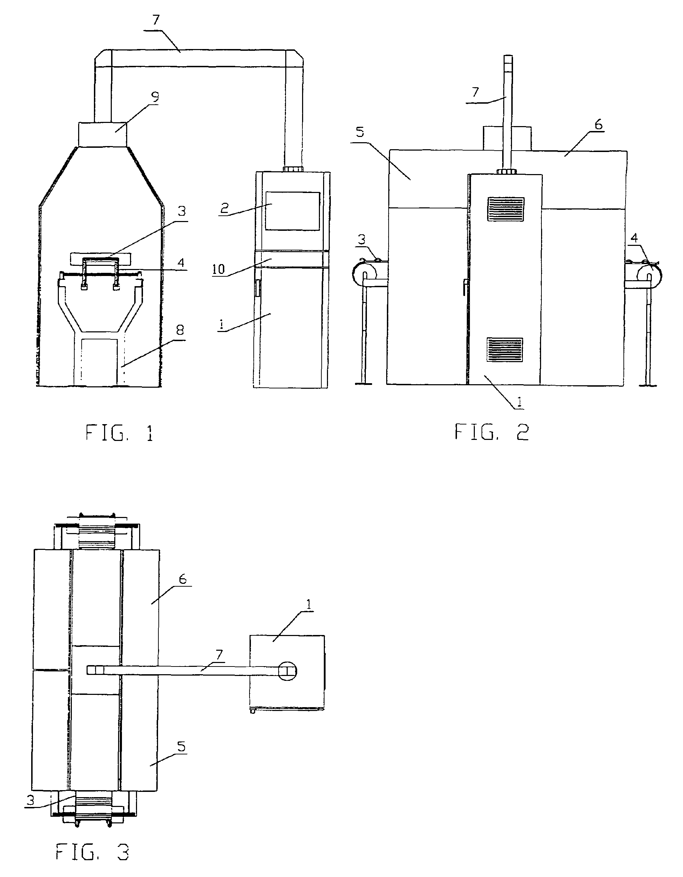

Figure 1 shows a front elevation view of the machine, with the control cabin (1), the computer monitor (2), the ceramic product (tile) (3), the drive pulleys (4), the casing for protection from ambient noise (5), the casing for protection from ambient light and dust (6), and cable bearing arm (7) which connects the control cabin to the control areas.
Figure 2 is a side view of the machine.
Figure 3 is a plan view of the machine.

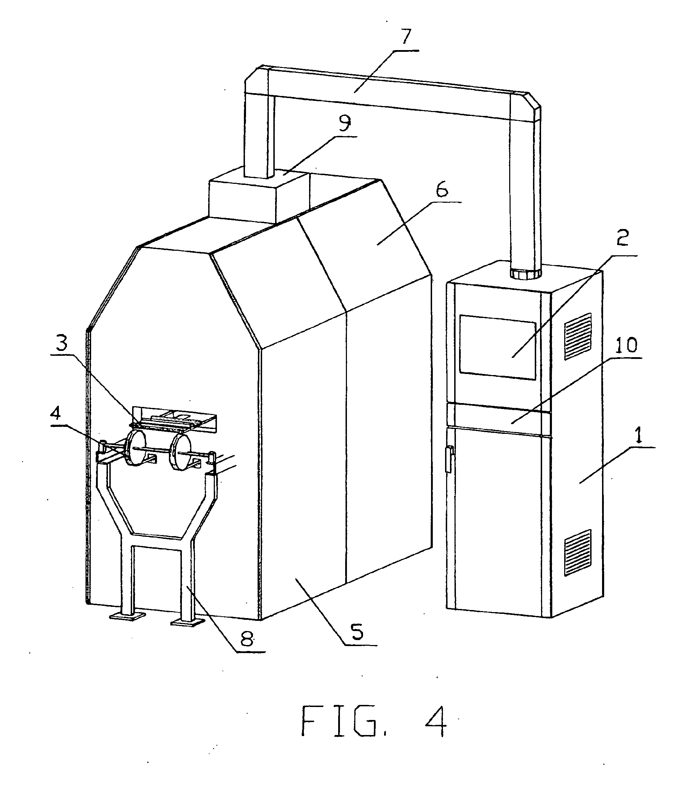

Figure 4 is a perspective view which is provided for a better comprehension and in which is shown the relative spatial arrangement of the various parts of the machine.

### PREFERRED EMBODIMENT OF THE INVENTION

The machine for automatic quality control of ceramic products comprises a mechanical mount (8) supporting the drive system, which consists of two belts which by means of a system of pulleys (4) are driven by a motorised reducer.

Said reducer is electrically connected to a frequency shifter which allows to change the speed of the belts and thereby the speed at which pass the parts to be controlled.

The machine comprises a first control area (5) in which the sound wave is obtained for each part using a hammer which strikes it automatically when the part crosses the area.

This wave is sent to the computer housed in cabin (1) for a frequency analysis.

The second area (6), where the artificial vision system is located, obtains an image of the part under inspection and sends it to the computer in cabin (1) for analysis.

Both sound area (5) and image area (6) are protected from dust by a protection case. In addition sound area (5) is soundproofed to prevent interference from ambient noise. Furthermore, image area (6) is pressurised to prevent entry of dust through the part passage openings.

Artificial vision area (6) is completed by a frontal lighting system and a backlighting system, both having a stabilised power source to avoid variations of the lighting intensity.

In addition, the cameras are positioned automatically. According to the type of part selected by the user by the computer onscreen menus, the system automatically positions the cameras through a linear actuator driven by a servomotor and a programmable robot.

The machine is also provided with a dust proof cabin pressurisation system and a chamber refrigeration system (9).

Control cabin (1) houses the computer, the monitor (2), the printer and the removable keyboard (10), as well as other elements required for the electronic control of the system, such as stabilised power sources, static relays, magnetothermal protection switches and a programmable robot.

The system is completed by an arm (7) which connects the control cabin to the areas (1) and (2) where the control and command cables are housed.

After analysing the sound wave and image of each part in the computer, the system classifies each part as either good or bad. If the part is bad the corresponding electrical signal is generated for expulsion of the part from the inspection line.

In addition, if the part is classified as bad the system will display the type of defect on the monitor:
- Paint defect
- Incorrect colour hue
- Broken corners or wrong angles
- Incomplete part
- Size outside tolerance limits
- Fissures

The monitor also has part counters which provide at all times the following information:
- Total number of parts controlled
- Total number of correct parts
- Total number of incorrect parts

In addition, there are other counters on the monitor screen which classify incorrect parts according to the type of defect, such as:
- Total number of parts lacking material
- Total number of parts with sound defects
- Total number of parts with colour defects
- Total number of parts with paint defects

The system can also print out work shift, daily, weekly or monthly historical and statistical records.

The screen also shows pie charts which tally the total number of good and bad parts, which are updated in real time as the parts pass.

In addition, the monitor displays in real time the image and outline of each part and its sound wave spectrogram.

In view of the above description and of the drawings the advantages derived from the system can be appreciated, compared to the traditional manual method for quality control of ceramic products.

The materials used to manufacture the components of the machine as well as their shape and size, and any other details which may be involved are considered independent of the object of the invention as long as the essence of the invention is not affected.

## Claims

1. Machine for automated quality control of ceramic products by artificial vision and sound analysis, comprising two control areas, the first area (5) in which the sound system is installed and the second area (6) in which the artificial vision system is installed, essentially **characterised in that** it performs a complementary sound and image analysis for detection of concealed or internal defects and external or visible defects respectively and it is provided with a system for protection from ambient noise, light and dust, which consists of protective cases (5) and (6), soundproofing (5) and pressurisation system (9) of the artificial vision area (6), as well as a refrigeration system for these chambers.

## Patentansprüche

1. Vorrichtung zur automatischen Qualitätskontrolle von Keramikprodukten durch künstliche Sicht- und Klanganalyse, umfassend zwei Kontrollbereiche, und zwar einen ersten Bereich (5), in dem das Klangkontrollsystem eingebaut ist und einen zweiten Bereich (6), in dem das künstliche Sichtkontrollsystem eingebaut ist, im Wesentlichen **dadurch gekennzeichnet, dass** sie eine einander ergänzende Bild- und Klanginspektion zur Aufdeckung versteckter bzw. innerer und sichtbarer bzw. äußerer Defekte durchführt und mit einem System zum Schutz vor Lärm, Licht und Staub aus der Umgebung ausgestattet ist, das aus Schutzgehäusen (5) und (6), einem Schallschutz- (5) und einem Überdrucksystem (9) für den Bereich (6) zur künstlichen Sichtkontrolle sowie einem Kühlsystem für diese Gehäuse besteht.

## Revendications

1. Appareil pour le contrôle automatique de la qualité des produits céramiques par une analyse visuelle et sonore artificielle, comprenant deux zones de contrôle, la première zone (5) dans laquelle est installé le système sonore et la seconde zone (6) dans laquelle est installé le système de vision artificielle, essentiellement **caractérisé en ce qu'**il exécute une analyse complémentaire du son et de l'image pour la détection de défauts cachés ou internes et de défauts externes ou visibles respectivement et **en ce qu'**il est pourvu d'un système de protection contre les bruits ambiants, la lumière et la poussière, qui est constitué de casiers protecteurs (5) et (6), d'une insonorisation (5) et d'un système de pressurisation (9) de la zone de vision artificielle (6), ainsi que d'un système de réfrigération pour ces chambres.
